# EUROPÄISCHE PATENTSCHRIFT

(11) **EP 0 470 393 B1**
(45) Veröffentlichungstag und Bekanntmachung des Hinweises auf die Patenterteilung: **19.10.1994**
(21) Anmeldenummer: 91111696.0
(22) Anmeldetag: 12.07.1991
(51) Int. Cl.: A61M 5/315

(54) **Granulatspritze**
Granules syringe
Séringue pour granulés

(30) Priorität: 10.08.1990 DE 9011685 U
(43) Veröffentlichungstag der Anmeldung: 12.02.1992
(73) Patentinhaber: THERA Patent GmbH & Co. KG Gesellschaft für industrielle Schutzrechte, D-82229 Seefeld (DE)
(72) Erfinder: Brandhorst, Gerd, W-8000 München 71 (DE); Täschner, Wolfgang, W-8913 Schondorf (DE)
(74) Vertreter: Strehl Schübel-Hopf Groening & Partner

(56) Entgegenhaltungen:
- EP-A- 0 170 120
- EP-A- 0 266 058
- EP-A- 0 275 363
- EP-A- 0 380 867
- US-A- 4 690 154
- Prospectus der Granulatspritze der Firma Orthomatrix

## Beschreibung

In der Chirurgie werden gelegentlich im Knochen entstandene Hohlräume mit einer Masse aufgefüllt, die aus einem mit dem Eigenblut des Patienten getränkten Knochenzementgranulat besteht. Für diesen Zweck ist eine Granulatspritze auf dem Markt, die die Merkmale gemäß dem ersten Teil des Anspruchs 1 aufweist.

Bei der bekannten Spritze ist das Granulat in einem durchsichtigen Röhrchen zwischen einer auf das vordere Röhrchenende aufgesteckten Kappe und einem in dem Röhrchen verschiebbaren Kolben angeordnet. Die Kappe ist in dem Bereich der Röhrchenöffnung siebartig ausgebildet, so daß die das Granulat tränkende Flüssigkeit hindurchtreten kann, das Granulat selbst aber zurückgehalten wird. Der Kolben läßt sich über eine am hinteren Röhrchenende herausgeführte Kolbenstange manuell verschieben.

Beim Einsatz wird die Granulatspritze mit der vorderen Kappe in das dem Patienten entnommene Blut eingetaucht und dieses durch Zurückziehen des Kolbens angesaugt. Um das Granulat mit dem eingetretenen Blut zu mischen, wird die Vorrichtung anschließend geschüttelt. Für eine effiziente Durchmischung muß das Volumen der Kammer größer sein als das Gesamtvolumen von Granulat und Blut; mit anderen Worten muß auch Luft angesaugt werden. Nach Fertigstellung des Gemisches muß diese Luft durch Vorschieben des Kolbens herausgedrückt werden. Erst dann kann das Gemisch durch weiteres Vorschieben des Kolbens in den Knochenhohlraum eingebracht werden.

Eine Schwierigkeit besteht bei der bekannten Granulatspritze darin, daß eine gründliche Durchtränkung des Granulats schwer zu erreichen ist. Die Wirksamkeit der manuellen Schüttelbewegung hängt unter anderem von Geschick und Geduld der Helferin und von dem durch Ansaugen von Luft vergrößerten Volumen der Schüttelkammer ab. Da die vordere Kappe für Blut durchlässig ist, sind ferner der Heftigkeit der Schüttelbewegung Grenzen gesetzt, falls nicht für einen weiteren Verschluß gesorgt wird.

Ein erheblicher weiterer Nachteil der bekannten Vorrichtung liegt darin, daß beim Schütteln Luft in das Gemisch eingebracht wird, die die Brauchbarkeit der fertigen Füllmasse verschlechtert und sich höchstens teilweise oder nur unter beträchtlichem Aufwand wieder entfernen läßt.

Aus EP-A-0 266 058 ist eine weitere Vorrichtung zur Herstellung eines mit Blut getränkten Knochenzementgranulats bekannt, die ein eine Kammer zur Aufnahme des Granulats bildendes zylindrisches Gehäuse und einen diese Kammer nach hinten abschließenden, zum Ausbringen des Gemisches kolbenartig vorschiebbaren, luftdurchlässigen Stopfen aufweist. Zum Tränken des Granulats mit Flüssigkeit wird dort eine zweite Spritze benötigt, die dabei mit der Kammer gekoppelt werden muß.

Der Erfindung liegt die Aufgabe zugrunde, eine Granulatspritze anzugeben, die eine wirksame Durchtränkung des Granulats unter gleichzeitiger Entlüftung des Gemisches gewährleistet und außerdem in der Handhabung einfacher ist.

Die erfindungsgemäße Lösung dieser Aufgabe ist im Anspruch 1 gekennzeichnet. Bei der danach gestalteten Granulatspritze wird die Tränkflüssigkeit beim Zurückziehen des Kolbens durch das Granulat hindurchgesaugt, ohne daß die das Granulat enthaltende Kammer vergrößert wird. Dadurch wird eine intensive Tränkung ohne Schüttelbewegung sichergestellt. Gleichzeitig wird die anfangs in dem Granulat enthaltene Luft entfernt.

Aus DE 37 01 190 A1 ist eine Vorrichtung zum Ansaugen oder Ausbringen von flüssigen oder pastösen Massen, auch Knochenzement, bekannt, bei der der Kolben an seinem Umfang mittels einer Lamellendichtung gegenüber dem Zylinder abgedichtet ist, so daß in die im Zylinder vorhandene Masse gelangte Luft durch eine hinter dem Kolben angeschlossene Unterdruckquelle abgesaugt werden kann. Gestaltung und Verwendung sind bei der bekannten Vorrichtung aber wesentlich anders als bei dem erfindungsgemäßen Gerät.

Weiterbildungen der Erfindung, die teils hinsichtlich der Herstellung, teils hinsichtlich der Anwendung der Vorrichtung Vorteile erbringen, sind in den Unteransprüchen gekennzeichnet.

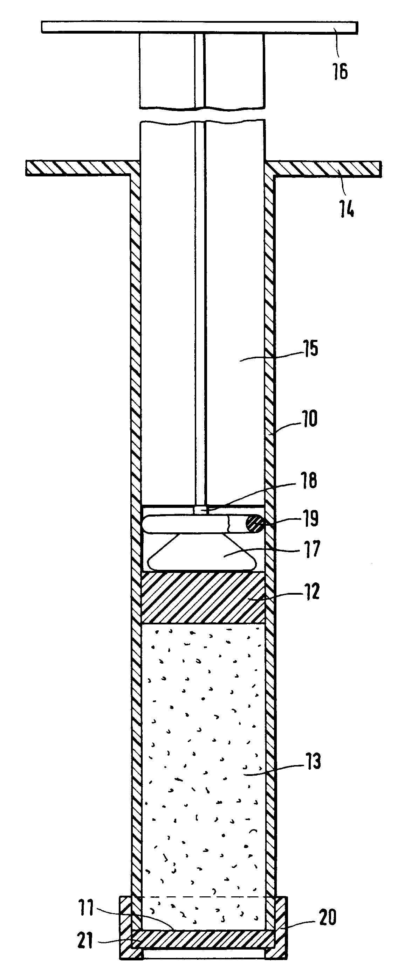

Ein bevorzugtes Ausführungsbeispiel der Erfindung wird nachstehend anhand der Zeichnung näher erläutert, die einen Längsschnitt durch eine Granulatspritze zeigt.

Gemäß der Zeichnung umfaßt die Granulatspritze ein vorzugsweise aus Glas oder durchsichtigem Kunststoff bestehendes röhrchenförmiges, zylindrisches Gehäuse 10 mit einer Länge von beispielsweise 120 mm und einem Außendurchmesser von beispielsweise 10 mm. In ihrem an die Ausbringöffnung 11 anschließenden, nach hinten von einem Stopfen 12 begrenzten vorderen Teil bildet das Gehäuse 10 eine Kammer zur Aufnahme von Granulat 13, das einen mittleren Teilchendurchmesser von beispielsweise 1 mm aufweist. An seinem von der Ausbringöffnung 11 abgewandten hinteren Ende ist das Gehäuse 10 offen und mit einem äußeren griffartigen Flansch 14 versehen.

Durch die hintere Öffnung ist eine Kolbenstange 15 eingeführt, die an ihrem hinteren Ende mit einer Griffscheibe 16 versehen ist und an ihrem vorderen Ende einen Kolbenkopf 17 trägt. Die Kolbenstange 15 ist so geformt, daß die zu entfernende Luft nach hinten entweichen kann. Hierzu sind z. B. sternförmige Querschnitte gut geeignet. In dem dargestellten Ausgangszustand liegt der Kolbenkopf 17 an der Rückseite des Stopfens 12 an. Die Kolbenstange 15 ist vorzugsweise so ausgelegt, daß bei maximalem Vorschub die Frontfläche des Stopfens 12 mit der vorderen Austrittsöffnung 11 des Gehäuses 10 bündig abschließt.

Der sich nach hinten konisch verjüngende Kolbenkopf 17 ist mit dem axial mittleren Teil der Kolbenstange 15 über einen axialen Zapfen 18 verbunden. Der Zapfen 18 durchsetzt einen Dichtungs-O-Ring 19, der mit seiner Außenkante an der Innenwand der zylindrischen Gehäuses 10 dichtend anliegt und sich beim Zurückziehen des Kolbens mit seiner inneren Öffnung dichtend an die sich konisch verjüngende hintere Fläche des Kolbenkopfes 17 anlegt. Beim Vorschieben des Kolbens entfernt sich der O-Ring 19 von dem Kolbenkopf 17, so daß durch seine innere Öffnung hindurch eine Luftdurchtrittsöffnung frei wird.

Der Stopfen 12 besteht aus porösem Material, beispielsweise gesintertem Polyethylen, mit einer Porengröße von beispielsweise 150 µm. Die Porosität ist so gewählt, daß zwar Luft hindurchtreten kann, das Granulat 13 jedoch nicht entweicht. Vorzugsweise kann die Porosität so gewählt sein, daß auch die zum Tränken des Granulats 13 verwendete Flüssigkeit, beispielsweise Blut, nicht ohne weiteres durch den Stopfen 12 hindurchtritt.

Auf das Austrittsende 11 des Gehäuses 10 ist im Ausgangszustand eine Kappe 20 aufgesteckt oder aufgeschraubt, die im Bereich der Austrittsöffnung 11 eine Scheibe 21 aus porösem Material aufweist. Bei dem Material kann es sich ähnlich wie bei dem Stopfen 12 um gesintertes Polyethylen handeln. Die Porengröße ist so gewählt, daß die Scheibe 21 für die Tränkflüssigkeit durchlässig, für das Granulat 13 dagegen undurchlässig ist.

Die beschriebene Granulatspritze wird vom Hersteller in dem in der Zeichnung gezeigten Zustand mit einer Füllung an Granulat 13 geliefert. Beim Einsatz wird diese Spritze mit aufgesetzter Kappe 20 in die Tränkflüssigkeit eingetaucht, die durch Zurückziehen der Kolbenstange 15 mit dem Kolbenkopf 17 und dem O-Ring 19 durch die in der Kappe 20 vorhandene poröse Scheibe 21 hindurch in das Granulat 13 angesaugt wird. Da das Gehäuse 10 durchsichtig ist, läßt sich das Eindringen der Flüssigkeit in das Granulat beobachten. Erreicht die Flüssigkeit den Stopfen 12, so wird die Rückziehbewegung des Kolbens beendet. In diesem Zustand ist das Granulat vollständig getränkt.

Der Kolben kann anschließend wieder in seine Ausgangsstellung eingeschoben werden, wobei die durch das Granulat und den Stopfen 12 hindurchgesaugte Luft durch die nun ventilartig freiwerdende innere Öffnung des O-Rings 19 hindurch nach hinten entweichen kann. Zum anschließenden Ausbringen des getränkten Granulats wird die Kappe 20 entfernt und der Stopfen 12 nun mit dem Kolbenkopf 17 nach vorne geschoben.

Der Stopfen 12 ist so dimensioniert, daß er in dem zylindrischen Gehäuse 10 mit genügender Festigkeit sitzt und sich beim Zurückziehen des Kolbens durch den zum Ansaugen der Flüssigkeit aufgebauten Unterdruck nicht nach hinten verschiebt. Eine derartige Verschiebung kann auch durch einen an der zylindrischen Innenwand des Gehäuses 10 hinter dem Stopfen 12 vorgesehenen Anschlag verhindert werden.

Die oben beschriebene, in den Kolben integrierte, von dem O-Ring 19, dem Kolbenkopf 17 und der Kolbenstange 15 gebildete ventilartige Dichtung ist nur ein Ausführungsbeispiel. Eine beim Zurückziehen des Kolbens geschlossene und beim Vorschieben sich öffnende Dichtung läßt sich alternativ durch entsprechende Gestaltung einer Dichtlippe erreichen. Ferner ließe sich die gewünschte Wirkung durch eine Ventilanordnung in der Wand des Gehäuses 10 hinter dem Stopfen 12 erreichen. Auch der zur Erzielung einer nach hinten durchgehenden Öffnung vorgesehene sternförmige Querschnitt der Kolbenstange 15 stellt nur ein Ausführungsbeispiel dar.

Die oben beschriebene Granulatspritze ist, ohne auf dieses Anwendungsgebiet beschränkt zu sein, in erster Linie zum Vorbereiten und Ausbringen von mit Eigenblut eines Patienten getränktem Knochenzementgranulat zum Auffüllen von Knochenhohlräumen bestimmt.

## Patentansprüche

1. Granulatspritze zum Tränken von Granulat mit einer Flüssigkeit und Ausbringen des Gemisches, umfassend
ein eine Kammer zur Aufnahme des Granulats (13) bildendes zylindrisches Gehäuse (10) mit einer vorderen Ausbringöffnung (11),
eine die Kammer nach vorne begrenzende, für die Flüssigkeit durchlässige, abnehmbare Abdeckung (21) und
einen die Kammer nach hinten abschließenden, zum Ausbringen des Gemisches kolbenartig vorschiebbaren Stopfen (12),
dadurch gekennzeichnet,
daß der Stopfen (12) luftdurchlässig ist,
daß hinter dem Stopfen (12) ein von diesem getrennter Kolben (15, 17) verschiebbar angeordnet ist, und
daß ebenfalls hinter dem Stopfen (12) ein beim Zurückziehen des Kolbens (15, 17) geschlossenes und beim Vorschieben öffnendes Ventil vorgesehen ist.

2. Granulatspritze nach Anspruch 1, dadurch gekennzeichnet, daß das Ventil in die Kolbendichtung integriert ist.

3. Granulatspritze nach Anspruch 2, dadurch gekennzeichnet, daß die Kolbendichtung aus einem an der zylindrischen Gehäusewand anliegenden O-Ring (19) besteht, der sich beim Zurückziehen des Kolbens (15, 17) gegen einen an einem vorderen Kolbenkopf (17) ausgebildeten Dichtungssitz legt und beim Vorschieben eine den hinteren Kolbenteil (15) durchsetzende Öffnung freigibt.

4. Granulatspritze nach einem der Ansprüche 1 bis 3, dadurch gekennzeichnet, daß das Gehäuse (10) aus durchsichtigem Werkstoff besteht.

5. Granulatspritze nach einem der Ansprüche 1 bis 4, dadurch gekennzeichnet, daß die Abdeckung (21) in einer am Ausbringende (11) des Gehäuses (10) befestigbaren Kappe (20) enthalten ist.

## Claims

1. A granules syringe for impregnating granules with a liquid and dispensing the mixture, comprising
a cylindrical housing (10) forming a chamber for receiving the granules (13) and having a front dispensing opening (11),
a removable cover (21) defining a front limitation of the chamber and being permeable to the liquid, and
a plug (12) rearwardly closing the chamber and being adapted to be advanced in piston-like fashion for dispensing the mixture,
**characterised** in
that the plug (12) is air-permeable,
that a piston (15, 17), which is separate from the plug (12), is movably disposed behind the latter, and
that a valve is provided also behind the plug (12), the valve being closed when the piston (15, 17) is retracted and opened when the piston is advanced.

2. The granules syringe of claim 1, characterised in that the valve is integrated in the piston seal.

3. The granules syringe of claim 2, characterised in that the piston seal consists of an O-ring (19) engaging the cylindrical housing wall, the O-ring abutting a sealing seat formed at a front piston head (17) when the piston (15, 17) is retracted, and releasing an opening penetrating the rear portion (15) of the piston when the latter is advanced.

4. The granules syringe of any one of claims 1 to 3, characterised in that the housing (10) consists of transparent material.

5. The granules syringe of any one of claims 1 to 4, characterised in that the cover (21) is contained in a cap (20) which is adapted to be fixed to the dispensing end (11) of the housing (10).

## Revendications

1. Seringue pour granulés pour l'imprégnation de granulés avec un liquide et l'expulsion du mélange, comprenant un corps (10) de forme cylindrique formant une chambre (13) pour la réception des granulés et présentant une ouverture de distribution (11) antérieure, un couvercle (21) amovible qui délimite la chambre vers l'avant et laisse passer le liquide, et un bouchon (12) qui assure la fermeture de la chambre vers l'arrière et peut être déplacé vers l'avant, à la manière d'un piston, pour l'expulsion du mélange, caractérisée en ce que le bouchon (12) est perméable à l'air, qu'à l'arrière du bouchon (12) est disposé de manière mobile un piston (15, 17) séparé dudit bouchon, et que, également à l'arrière du bouchon (12), est prévue une soupape qui est fermée lorsque le piston (15, 17) est tiré en arrière et qui s'ouvre lors de l'avancement.

2. Seringue pour granulés selon la revendication 1, caractérisée en ce que la soupape est intégrée dans le joint d'étanchéité du piston.

3. Seringue pour granulés selon la revendication 2, caractérisée en ce que le joint d'étanchéité du piston est constitué par un joint torique (19) appliqué contre la paroi du corps cylindrique, lequel s'applique lors du retrait du piston (15, 17) contre un siège d'étanchéité conformé sur une tête antérieure (17) du piston et libère lors de l'avancement, une ouverture ménagée dans la partie postérieure (15) du piston.

4. Seringue pour granulés selon l'une des revendications 1 à 3, caractérisée en ce que le corps (10) est constitué d'un matériau translucide.

5. Seringue pour granulés selon l'une des revendications 1 à 4, caractérisée en ce que le couvercle (21) est logé dans un capuchon (20) qui peut être fixé sur l'extrémité de distribution (11) du corps (10).
